# EUROPEAN PATENT APPLICATION

(11) **EP 3 919 086 A2**
(43) Date of publication of application: **08.12.2021**
(21) Application number: 21177534.1
(22) Date of filing: 03.06.2021
(51) Int. Cl.: A61L 2/20, A61L 2/28

(54) **PORTABLE APPARATUS FOR SANITIZING ARTICLES**

(30) Priority: 03.06.2020 IT 202000013114
(71) Applicant: Steelco S.p.A., 31039 Riese Pio X (IT)
(72) Inventor: Zardini, Fabio, 31033 Castelfranco Veneto (TV) (IT)
(74) Representative: Petraz, Gilberto Luigi

(57) **Abstract**

Portable apparatus (10) for sanitizing articles (100) comprising a support frame (11) with which there are associated a container (12) for containing said articles (100), a circuit (13) for introducing a sanitizing fluid (SF), a circuit (17) for evacuating exhausted fluid (EF) from said container (12), and a fluidic movement assembly (19) associated with said introduction circuit (13) and with said evacuation circuit (17).

## Description

### FIELD OF THE INVENTION

The present invention concerns a portable apparatus for sanitizing articles, above all of small or medium size that need to be used several times.

The apparatus can be used in the medical, outpatient, dental, ophthalmic fields, and in general in all areas and sectors in which determinate reusable articles come into contact with, or are worn or used by, different people.

The apparatus can also advantageously be used for the initial sanitizing of articles that are not passed from one person to another, such as for example in the case of artisan shops that sell directly to the public.

The apparatus in question can favorably carry out a low-temperature sanitization treatment even of articles made of temperature-sensitive materials.

### BACKGROUND OF THE INVENTION

It is known that the surface of articles and objects is very often a vehicle for pathogens such as viruses and bacteria. The risk of transmitting a disease increases especially if these articles are reused or in any case handled or worn even several times and by different people.

In the pharmaceutical, hospital and outpatient sectors there are machines for sanitizing objects such as surgical instruments or other, which are typically very expensive and large.

Such known machines are normally provided with a large sanitizing chamber in which the objects to be treated are placed. Devices for conveying/discharging sanitizing fluids can be connected to the sanitizing chamber.

The sanitizing treatments in question can contemplate using, possibly also in a combined manner, chemical substances, detergents and water vapor, and operating at high temperature and pressure conditions and for defined periods of time. These operating conditions can possibly be subjected to specific regulatory requirements. Examples of sanitizing treatments can include pre-washing, washing, with water and possible chemical agents, both cold and also hot, thermal disinfection, possible drying and sterilization, the latter also hot, that is, with water vapor, or cold.

In some known solutions, the devices for conveying/discharging fluids can be used for example, but not only, to generate a condition of vacuum in the machine, aimed at obtaining the correct conditions for the sanitizing agent.

The conveying/discharging devices can also be used to introduce a washing liquid to wash the objects disposed in the treatment chamber before their sanitization.

A device for feeding a fluid substance that is functional to the sterilization and/or washing steps as above can also be fluidically connected to the sanitizing chamber.

The fluid substance used can be, for example, hydrogen peroxide or peracetic acid, or other chemical agents. The fluid substance is typically contained in a container, or cartridge, possibly associated with a dispenser, to be inserted in the machine before the start of a work cycle.

Some disadvantages of these machines are high costs, both to purchase and also manage them, the overall size and the need to install them in dedicated spaces. Furthermore, the machines as above are difficult to manage for unqualified personnel, complicated to position and reposition if the spaces of a building are redesigned or in the event of relocation. Furthermore, they often require connections to the water mains or to a system for discharging the fluids used in the process. All these limits prevent them from being used in more everyday situations, for example but not only in the retail sector.

Particularly in the context of the sale of wearable accessories or articles, such as glasses, watches, necklaces, which need to be tried on to confirm size or wearability, or in all those sectors in which certain objects are hired and therefore come into contact with many people, the need to make these articles safe in order to minimize the transmission of diseases, especially of a viral and bacterial nature, has highlighted the lack of specific apparatuses suited to this purpose. Those currently on the market, in fact, either do not guarantee an adequate degree of safety or tend to degrade or destroy the articles subjected to sanitizing.

For example, sanitizing apparatuses for small objects are known, which use UV-C rays technology, that is, rays having a wavelength in the range of 200-250nm, which are particularly suitable to act on the DNA of bacteria, viruses, germs, mites and other pathogenic microorganisms by eliminating their ability to reproduce.

However, after a certain number of treatment cycles articles made of plastic material or that have parts in plastic material tend to degrade and can no longer be used. This causes them to be continuously replaced, which translates into increased costs, but above all excludes a wide range of articles such as eyewear, sports watches, bracelets and identification badges, audio devices for museum visitors, and many others.

Another limitation consists in the fact that many objects have shapes or recesses that are difficult for UV-C lamps to irradiate directly.

Furthermore, these solutions have the disadvantage that ultraviolet rays - which can be very harmful - can hit the user with consequent risks to his/her health.

Other known apparatuses use, as indicated above, high temperature sanitizing technologies such as thermal disinfection or hot steam sterilization, and are not applicable for a wide range of articles by virtue of the nature of the materials that they are made of.

There is therefore the need to perfect a portable device for sanitizing articles that can overcome at least one of the disadvantages of the state of the art.

In particular, one purpose of the present invention is to provide a portable apparatus for sanitizing articles that is compact and space-saving, easy to install and move.

Another purpose of the present invention is to provide a portable apparatus for sanitizing articles that is inexpensive and that is made with mechanically simple components, which are easy to repair in the event of a failure and economical if they need replacing.

Another purpose of the present invention is to provide a portable apparatus for sanitizing articles that guarantees a high degree of reliability of the sanitized articles, and possibly also provides proof, or certainty, that sanitization has occurred, that is, that the sanitization process has been successful.

The Applicant has devised, tested and embodied the present invention to overcome the shortcomings of the state of the art and to obtain these and other purposes and advantages.

### SUMMARY OF THE INVENTION

The present invention is set forth and characterized in the independent claim.

The dependent claims describe other characteristics of the present invention or variants to the main inventive idea.

In accordance with the above purposes, a portable apparatus for sanitizing articles, which overcomes the limits of the state of the art and eliminates the defects present therein, comprises:
- a fluid-tight container for containing one or more articles to be sanitized,
- a source of sanitizing fluid provided with means for introducing one or more sanitizing agents,
- a circuit for introducing a sanitizing fluid into the container provided with conditioning means able to convert the one or more sanitizing agents into a flow of sanitizing fluid able to enrich the inside of the container,
- a circuit for evacuating exhausted fluid from the container,
- a fluidic movement assembly connected to the introduction circuit and to the evacuation circuit.

In particular, the container, the source of sanitizing fluid, the introduction circuit, the evacuation circuit and the fluidic movement assembly are supported by a common support frame.

### BRIEF DESCRIPTION OF THE DRAWINGS

These and other aspects, characteristics and advantages of the present invention will become apparent from the following description of some embodiments, given as a non-restrictive example with reference to the attached drawings wherein:
- fig. 1 schematically shows a portable apparatus for sanitizing articles in accordance with some embodiments described here;
- figs. 2-4 schematically show some possible variants of the apparatus of fig. 1.

To facilitate comprehension, the same reference numbers have been used, where possible, to identify identical common elements in the drawings. It is understood that elements and characteristics of one embodiment can conveniently be combined or incorporated into other embodiments without further clarifications.

### DETAILED DESCRIPTION OF SOME EMBODIMENTS

We will now refer in detail to the possible embodiments of the invention, of which one or more examples are shown in the attached drawings, by way of a non-limiting illustration. The phraseology and terminology used here is also for the purposes of providing non-limiting examples.

Some embodiments described here concern a portable apparatus 10 for sanitizing articles 100, preferably of small and medium size.

By way of example only, the articles or objects in question can be of the most varied kind, for example medical devices and instruments, jewelry, watches, necklaces, glasses, or other. All such articles 100 are preferably of the type that can be reused, often by different people, and therefore need to be sanitized.

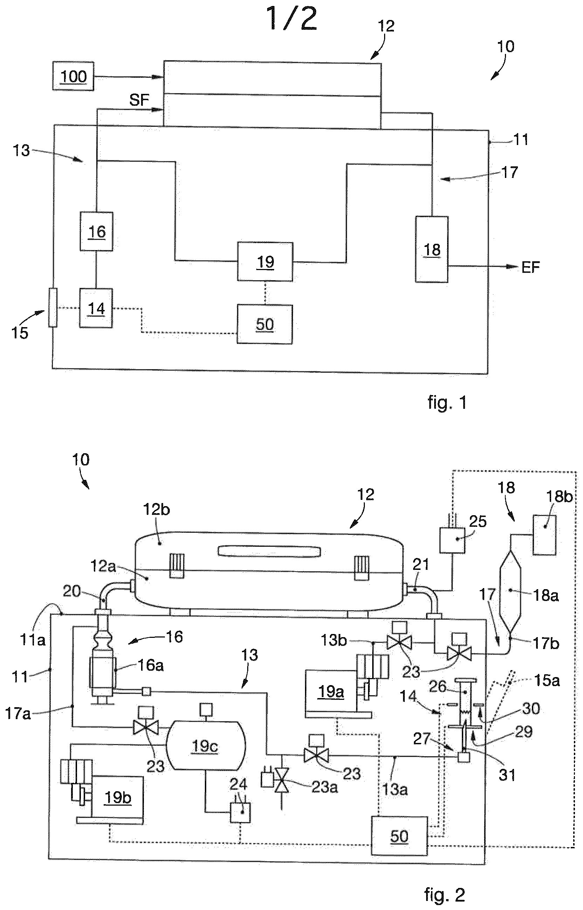

With particular reference to the example diagram of fig. 1, the apparatus 10 comprises a support frame 11 with which there is associated a container 12 that can be selectively opened and hermetically closed in order to temporarily contain the articles 100 as above.

With the support frame 11 there is also associated a circuit 13 for introducing a sanitizing fluid SF into the container 12, and a source of sanitizing fluid 14 provided with means 15 for introducing the sanitizing fluid SF and with conditioning means 16 able to generate a flow of sanitizing fluid SF, starting from one or more sanitizing agents, in order to enrich the inside of the container 12. In particular, the conditioning means 16 are disposed between the source of sanitizing fluid 14 and the container 12.

With the support frame 11 there is also associated a circuit 17 for evacuating exhausted fluid EF from the container 12 which, in this specific case, comprises abatement means 18 for abating the exhausted fluid before it is introduced into the atmosphere. By exhausted fluid EF, here and hereafter in the description we mean the excess amount of sanitizing fluid SF, that is, which has not reacted, or has not come into contact, with the surfaces of articles 100.

The apparatus 10 comprises a fluidic movement assembly 19 also associated with the support frame 11 and fluidically connected to the introduction circuit and the evacuation circuit 17.

According to some embodiments, the support frame 11 has a box-like structure and has an upper surface 11a onto which the container 12 is attached, possibly in a removable manner.

The support frame 11 can be provided with support feet, for its stable positioning on a plane, for example a counter, a shelf, a table, or it can be trolley mounted to allow it to be easily moved by dragging.

The support frame 11 houses inside it the introduction circuit 13 and the evacuation circuit 17 which are therefore totally invisible from the outside except for the presence of a lateral door 15a for accessing the introduction means 15, as will be better described below. All the components therefore remain contained inside the support frame 11. This allows to obtain a very compact apparatus 10, without possible elements that can be tampered with or exposed in a way that is dangerous for the user.

The support frame 11 has connectors 20, 21 and possibly 22 that connect the container 12 to the introduction circuit 13 and to the evacuation circuit 17.

According to some embodiments, the container 12 can be closed hermetically and internally delimits a housing compartment to place, in a condition protected from the outside, the articles 100 to be sanitized.

The container 12 is formed by at least two parts 12a, 12b associated with each other, of which a first bottom part 12a, where the articles 100 can be rested, and a second closing part 12b, which are selectively mobile with respect to each other between a closing condition, in which the second part 12b completely covers the first part 12a protecting the articles inside the housing compartment, and an opening condition, in which the introduction of the articles 100 to be sanitized is allowed.

The container 12 has, favorably in the bottom part 12a, watertight flanges to receive the connectors 20, 21 and possibly 22.

In one possible embodiment, the container 12 can be a hospital-type bag provided with a hermetically sealed aperture for introducing the articles 100 and with entry and exit connectors for connection with the introduction circuit 13 and the evacuation circuit 17. The container 12 can also be used to preserve the sanitized articles 100 and has to be replaced at each sanitization cycle. In this case, the apparatus 10 can comprise sealing means for the hermetic closure of the bag.

According to some embodiments, the fluidic movement assembly 19 can be used to generate a vacuum condition in the container 12, aimed at promoting the introduction of the sanitizing fluid SF and to allow the evacuation of the exhausted fluid EF.

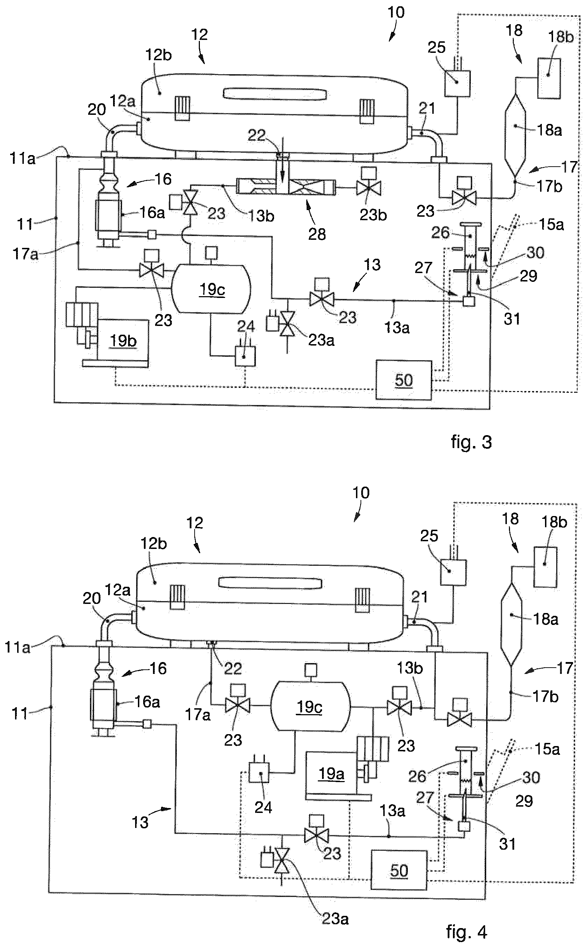

Depending on the circuit configurations, the fluidic movement assembly 19 can comprise a first fluidic movement device 19a, in a vacuum pump and compressor configuration (fig. 4), or a second fluidic movement device 19b in a compressor combined with a Venturi device 28 configuration (fig. 3), or both the fluidic movement devices 19a, 19b, as shown in the example of fig. 2.

In the event that only one of the fluidic movement devices 19a, 19b is present, it is used both to generate the vacuum in order to recall the sanitizing fluid SF into the container 12, and also to allow the evacuation of the exhausted fluid EF. Obviously, using only one of the fluidic movement devices 19a, 19b allows to reduce the number of components required and the overall cost of the apparatus 10.

In the event that the fluidic movement devices 19a, 19b are used only, or also, in a compressor configuration, the presence of a tank 19c is provided with which there is associated a pressure measuring device configured as a pressure switch 24. The pressure switch 24 serves to control and verify the pressure of the flow of air that is introduced into the container 12 to evacuate the exhausted fluid EF.

The air sucked in from the external environment to evacuate the exhausted fluid EF can require a specific treatment, since the articles 100 are still inside the container 12 and are hit by the flow of air. For this purpose, the apparatus 10 can be equipped with an abatement filter, for example of the HEPA type, able to generate a substantially sterile flow of air.

According to the embodiment of fig. 3, in which only the second movement device 19b is present in the compressor configuration, the presence of the Venturi device 28 is provided, fluidically associated with the tank 19c, with the container 12 and with the external environment. This device allows to generate the vacuum in the container 12 by Venturi effect. The Venturi device 28 can be provided, at exit, with a Clapet valve 23b.

Depending on the presence or absence of one or both of the fluidic movement devices 19a, 19b, the introduction circuit 13 and the evacuation circuit 17 can have different structures and derivations which a person of skill in the art can easily derive and which are susceptible to possible variants.

In the example described in figs. 2-4, the first circuit 13 comprises a first duct 13a, which fluidically connects the source of sanitizing fluid 14 with the conditioning means 16. The conditioning means 16 are connected to the container 12 by means of another duct ending with a first connector 20.

With reference to fig. 2 and fig. 4, the introduction circuit 13 comprises a second duct 13b which fluidically connects the container 12, by means of a second connector 21, to the first movement device 19a.

In the example of fig. 3, where only the second movement device 19b configured as a compressor is present, the first circuit 13 comprises a second duct 13b which fluidically connects the tank 19c with the Venturi device 28. The Venturi device 28 can be connected to the container 12 by means of a third connector 22 and has an exit for the flow of compressed air arriving from the first movement device 19b.

According to some embodiments, with the second duct 13b there can be associated a pressure measuring device configured as a vacuum switch 25. The vacuum switch 25 serves to control and verify the vacuum level inside the container 12. The vacuum switch 25 can also be used to check the presence of sanitizing fluid SF inside the container 12. In fact, once the required vacuum condition is reached, if the sanitizing fluid SF is introduced into the chamber, the pressure increases more than the increase caused by introducing, for example, only air. This can be used as an indicator of successful sanitation. Optionally, the vacuum switch 25 can be programmed to send a signal of successful sanitation to control means 50 of the apparatus 10. Such control means 50 can include, for example, a microcontroller, an industrial PC or a PLC (Programmable Logic Controller).

In the example described in figs. 2-4, the evacuation circuit 17 comprises a second duct 17b which fluidically connects the container 12 to the outside, by means of the second connector 21. The abatement means 18 are associated with the second duct 17b.

According to some embodiments, the abatement means 18 can comprise a catalytic filter 18a and/or an active carbon filter 18b.

In possible embodiments, the abatement means 18 can provide abatement cells suitably heated to abate the content of particular sanitizing gases, for example ozone. This heating can be generated by a heat pump or by a simple heating resistance.

According to possible embodiments, the abatement filters or cells can be made with particular materials which can change morphology or color with the passage of the exhausted fluid EF. The filters or cells can provide a control window through which the user can verify the effectiveness of the abatement effect, but above all verify that the abated sanitizing fluid has exited, which would confirm that the sanitization cycle has been successfully completed.

According to possible embodiments, in the event that the sanitizing treatment is carried out with a sanitizing fluid SF which produces an exhausted fluid EF that does not need abating, the abatement means 18 may not be present.

With reference to figs. 2-3, the evacuation circuit 17 comprises a first duct 17a, which fluidically connects the second movement device 19b, in particular the tank 19c, with the container 12 in correspondence with the first connector 20.

In the example of fig. 4, the first duct 17a is fluidically associated with the first movement device 19a and with the container 12.

According to some embodiments, the first ducts 13a, 17a and the second ducts 13b, 17b comprise respective valves 23 for intercepting the flow. Such valves can preferably be solenoid valves. Depending on the step of the sanitization cycle, the valves 23 can be opened or closed to isolate determinate parts of the circuits 13, 17.

According to some embodiments, the first duct 13a can be provided with a draining valve 23a to eliminate the sanitizing fluid SF present in that part of the circuit, for example in the case of maintenance.

According to some embodiments, the sanitizing fluid SF can be a fluid substance, for example liquid or gaseous such as hydrogen peroxide, peracetic acid, or other fluid substances suitable for the purpose. Optionally, the sanitizing fluid SF can be obtained starting from one or more sanitizing agents.

According to possible embodiments, the sanitizing fluid SF can be ozone. In this case, the source of the sanitizing fluid 14 can be directly the environment from which the air is drawn, while the conditioning means 16 can be configured as an ozone generator essentially of a known type.

Preferably, the sanitizing fluid SF is hydrogen peroxide with a concentration higher than 50%.

The source of sanitizing fluid 14 can provide to supply sanitizing fluid in liquid form or directly in gaseous form.

In the event that the sanitizing fluid 14 is provided in liquid form, the conditioning means 16 can comprise a vaporizer device 16a inside which a coil passes, with the sanitizing fluid SF liquid being heated by means of a resistance. At exit, a sanitizing fluid SF is obtained in gaseous form which is then drawn thanks to the pressure difference present inside the container 12. In the event that the sanitizing fluid SF is hydrogen peroxide, the temperature of the resistance is comprised between about 85°C and 105°C, preferably about 90°C. In fact, at temperatures above about 100°C, the Applicant has experienced phenomena of crystallization of the hydrogen peroxide.

According to some embodiments, in the event that the sanitizing fluid SF is supplied in the liquid state, the fluid substance is typically contained in at least one cartridge 26 of the disposable type, to be inserted in the apparatus 10 before the start of a work cycle and to be replaced at the end.

According to some embodiments, the at least one cartridge 26 can be filled with a quantity of liquid comprised between about 0.3ml and about 3ml, preferably about 1ml.

In this case, the introduction means 15 can comprise a support body provided with an introduction seating in which, during use, at least one cartridge 26 is disposed. The introduction seating can be selectively accessible from a door 15a, shown with a dashed line in figs. 2-4.

To the support body there is connected a unit 27 for removing the fluid substance which generally comprises one or more perforating elements 31, for example hollow needles. The perforating elements 31 are disposed in a fixed position in the introduction seating, protruding, for example, from a bottom wall thereof.

During the introduction of at least one cartridge 26 into the introduction seating, the perforating elements 31 perforate the at least one cartridge 26 and allow the fluid substance to be removed. For this purpose, the cartridge 26 typically has at least one wall portion made of a pierceable material, for example rubber.

The fluid substance, passing through the perforating elements 31, is drawn into the first duct 13a, due to the pressure difference previously generated in the container 12, and passes into the vaporizer device 16a which prepares it to be drawn/introduced into the container 12.

According to some embodiments, the apparatus 10 comprises detection means 29 to verify the presence or absence of at least one cartridge 26. The detection means 29 can be associated with the support body and communicate with the introduction seating.

According to possible embodiments, the detection means 29 can be selected from a group comprising a mechanical micro switch, an optical sensor, a weight sensor or load cell. In the case of a weight sensor, this can also verify that the cartridge 26 is filled correctly, or it can verify the presence/absence of liquid inside it. In the case of an optical sensor and a weight sensor, it is possible to provide the presence of the control means 50 which receive the signal from the sensors and send a possible operating signal of activation/deactivation to the fluidic movement assembly 19.

According to some embodiments, the apparatus 10 can comprise anti-tampering means 30 configured to provide proof, or certainty, of completed sanitization.

The anti-tampering means 30 can be associated with the support body and communicate with the introduction seating in order to possibly cooperate with the at least one cartridge 26. In fact, the need to guarantee the sanitization of the articles 100 is strictly connected with the need to prevent tampering of the cartridges 26.

The anti-tampering means 30 can be of the mechanical type or can integrate more or less complex electronic sensors that require the presence of the control means 50 which receive control signals at input and process one or more signals at output in order to command, for example, the fluidic movement assembly 19 or to send an error signal to a graphic interface with the user.

According to possible embodiments, the anti-tampering means 30 can cause a predefined breakage of the cartridge 26 in order to make it no longer reusable once its function is completed. In this case, the anti-tampering means 30 can comprise cutting elements that sever the at least one cartridge 26, or they can comprise other perforation elements that completely, or almost completely, break through the portion of the wall made of pierceable material.

In other possible solutions, the anti-tampering means 30 can comprise a loading device, for example of the drum type, equipped with a predefined number of cartridges 26 that on each occasion are made unavailable once used.

According to other possible embodiments, the anti-tampering means 30 can provide at least one chute for discharging the empty cartridges 26. For example, the discharge chute can be provided in selective communication with the introduction seating. The cartridges 26 can then fall into a specific unloading zone, possibly accessible only to maintenance technicians with special access keys.

According to other possible embodiments, the anti-tampering means 30 can provide the presence on at least one cartridge 26 of an RFID label, and on the apparatus 10 of an RFID reading/writing device. When the at least one full cartridge 26 is inserted, the reading/writing device reads the information contained in the RFID label and, by means of the control means 50, sends an activation/deactivation signal for example to the fluidic movement assembly 19. At the end of the sanitization cycle, the reading/writing device is configured to write the new information of "empty cartridge" on the RFID label. In this way, it is impossible to reuse previously used cartridges 26.

According to other possible solutions, the anti-tampering means 30 can provide a litmus-type paper to be positioned inside the container 12 or to be attached to each article 100 to be sanitized, in a manner that cannot be tampered with. In this way, it is possible to verify and confirm that the sanitization process has taken place correctly.

According to some embodiments, the control means 50, depending on the configurations, are operatively associated with the fluidic movement devices 19a, 19b, with the pressure switch 24, with the vacuum switch 25, with the detection means 29 and with the anti-tampering means 30 in order to receive and/or send respective operating signals to activate/deactivate the components as above.

In possible implementations of the apparatus 10, it is possible that the latter is controlled remotely, directly by the manufacturer who can verify its correct functioning and use.

It is clear that modifications and/or additions of parts may be made to the portable apparatus for sanitizing articles as described heretofore, without departing from the field and scope of the present invention as defined by the claims.

In the following claims, the sole purpose of the references in brackets is to facilitate reading: they must not be considered as restrictive factors with regard to the field of protection claimed in the specific claims.

## Claims

1. Portable apparatus (10) for sanitizing articles (100), **characterized in that** said apparatus (10) comprises a support frame (11) which supports:
a fluid-tight container (12) for containing one or more articles (100) to be sanitized,
a source (14) of sanitizing fluid provided with means (15) for introducing one or more sanitizing agents,
a circuit (13) for introducing a sanitizing fluid (SF) into said container (12) provided with conditioning means (16) able to convert said one or more sanitizing agents into a flow of sanitizing fluid (SF) able to enrich the inside of said container (12),
a circuit (17) for evacuating exhausted fluid (EF) from said container (12),
a fluidic movement assembly (19) connected to said introduction circuit (13) and to said evacuation circuit (17);
**and in that** the introduction circuit (13), the evacuation circuit (17) and the fluidic movement assembly (19) are completely contained inside, or in the overall bulk of, said support frame (11).

2. Apparatus (10) as in claim 1, **characterized in that** it also comprises anti-tampering means (30) of the mechanical type or integrating electronic sensors, and configured to provide proof, or certainty, of completed sanitization.

3. Apparatus (10) as in claim 1 or 2, **characterized in that** said source (14) of sanitizing fluid is a cartridge (26) of the disposable type pre-filled with a defined quantity of sanitizing fluid (SF).

4. Apparatus (10) as in claim 2 and 3, **characterized in that** said anti-tampering means (30) of the mechanical type comprise elements able to irreversibly change the physical conformation of said at least one cartridge (26) to make it non-reusable.

5. Apparatus (10) as in claim 4, **characterized in that** said anti-tampering means (30) comprise cutting elements which sever the at least one cartridge (26), or other perforation elements which completely, or almost completely, break through a portion of wall of the cartridge (26) made of pierceable material.

6. Apparatus (10) as in claim 3, 4 or 5, **characterized in that** said introduction means (15) comprise a support body in which said at least one cartridge (26) is able to be housed, said support body in particular being provided with an introduction seating in which, during use, at least one cartridge (26) is disposed, and a removal unit (27) comprising one or more perforating elements (31) able to perforate the at least one cartridge (26), wherein said anti-tampering means (30) are associated with the support body and communicate with the introduction seating in order to cooperate with the at least one cartridge (26).

7. Apparatus (10) as in any claim from 2 to 6, **characterized in that** said anti-tampering means (30) comprise a loading device equipped with a predefined number of cartridges (26) which on each occasion are made unavailable once used.

8. Apparatus (10) as in any claim from 2 to 7, **characterized in that** said anti-tampering means (30) comprise an unloading chute for unloading the empty cartridges (26), said unloading chute in particular being provided in selective communication with the introduction seating.

9. Apparatus (10) as in any claim from 2 to 8, **characterized in that** said anti-tampering means (30) comprise an RFID tag disposed on the cartridge (26) and an RFID reading/writing device on the apparatus (10).

10. Apparatus (10) as in any claim from 2 to 9, **characterized in that** said anti-tampering means (30) comprise a litmus paper to be positioned inside the container (12) or to be attached, in a manner that cannot be tampered with, to each article (100) to be sanitized.

11. Apparatus (10) as in any claim from 3 to 10, **characterized in that** it comprises detection means (29) to verify the presence or absence of said at least one cartridge (26).

12. Apparatus (10) as in any claim hereinbefore, **characterized in that** said sanitizing fluid (SF) is hydrogen peroxide.

13. Apparatus (10) as in any claim hereinbefore, **characterized in that** the conditioning means (16) comprise a vaporizer device (16a), disposed between the source (14) of sanitizing fluid and the container (12), and configured to vaporize the liquid of sanitizing fluid (SF).

14. Apparatus (10) as in any previous claim from 1 to 11, **characterized in that** the sanitizing fluid (SF) is ozone and the conditioning means (16) are configured as an ozone generator.

15. Apparatus (10) as in any claim hereinbefore, **characterized in that** said evacuation circuit (17) is provided with abatement means (18) that comprise catalytic filters (18a) and/or active carbon filters (18b).

16. Apparatus (10) as in any claim hereinbefore, **characterized in that** the fluidic movement assembly (19) comprises a first fluidic movement device (19a) associated with said introduction circuit (13) configured as a vacuum pump, and a second fluidic movement device (19b) associated with said evacuation circuit (17) configured as a compressor.

17. Apparatus (10) as in any claim from 1 to 14, **characterized in that** said fluidic movement assembly (19) comprises a first fluidic movement device (19a) in a configuration alternatively of vacuum pump or compressor associated both with said introduction circuit (13) and also with said evacuation circuit (17).

18. Apparatus (10) as in any claim from 1 to 14, **characterized in that** said fluidic movement assembly (19) comprises a second fluidic movement device (19b) configured alternatively as a compressor to allow the evacuation of the exhaust fluid (EF) and associated with a Venturi device (28) to generate a vacuum condition in the container (12).

19. Apparatus (10) as in any claim hereinbefore, **characterized in that** said container (12) is formed by at least two parts (12a, 12b) associated with each other, of which a first bottom part (12a), where the articles (100) to be sanitized are rested, and a second closing part (12b), which are selectively mobile with respect to each other between a closing condition, in which the second part (12b) completely covers the first part (12a) protecting the articles (100) inside a housing compartment, and an opening condition, in which the introduction of said articles (100) to be sanitized is allowed.
